# FASCICULE DE BREVET EUROPEEN

(11) **EP 3 256 568 B1**
(45) Date de publication et mention de la délivrance du brevet: **05.04.2023**
(21) Numéro de dépôt: 16705032.7
(22) Date de dépôt: 10.02.2016
(51) Int. Cl.: C12N 5/071, C12N 5/09

(54) **PROCEDE D'IMPLANTATION D'AMAS CELLULAIRES DANS UN EXPLANT DE PEAU**
VERFAHREN ZUM IMPLANTIEREN VON ZELLKLUMPEN IN EINEM HAUTEXPLANTAT
METHOD FOR IMPLANTING CELL CLUMPS IN A SKIN EXPLANT

(30) Priorité: 10.02.2015 FR 1500262
(43) Date de publication de la demande: 20.12.2017
(73) Titulaire: Genoskin, 31000 Toulouse (FR); Centre National de la Recherche Scientifique (C.N.R.S.), 75016 Paris (FR); Université Toulouse III-Paul Sabatier, 31062 Toulouse Cedex 9 (FR); CHU de Toulouse, 31059 Toulouse Cedex 9 (FR)
(72) Inventeur: JARDET, Claire, 31270 Cugnaux (FR); DESCARGUES, Pascal, 31500 Toulouse (FR); LOBJOIS, Valérie, 32600 Pujaudran (FR); DUCOMMUN, Bernard, 31450 Belderaud (FR)
(74) Mandataire: Barbot, Willy
(86) Numéro de dépôt international: PCT/EP2016/000223
(87) Numéro de publication internationale: WO 2016/128135

(56) Documents cités:
- WO-A1-2013/164436
- KRUGLUGER WALTER ET AL: "Reorganization of hair follicles in human skin organ culture induced by cultured human follicle-derived cells", EXPERIMENTAL DERMATOLOGY, BLACKWELL MUNSGAARD, COPENHAGEN; DK, vol. 14, no. 8, 1 août 2005 (2005-08-01), pages 580-585, XP002548419, ISSN: 0906-6705 [extrait le 2005-03-09]
- F. MEIER ET AL: "Combined targeting of MAPK and AKT signalling pathways is a promising strategy for melanoma treatment", BRITISH JOURNAL OF DERMATOLOGY, vol. 156, no. 6, 1 juin 2007 (2007-06-01), pages 1204-1213, XP055082399, ISSN: 0007-0963, DOI: 10.1111/j.1365-2133.2007.07821.x
- H VÖRSMANN ET AL: "Development of a human three-dimensional organotypic skin-melanoma spheroid model for in vitro drug testing", CELL DEATH AND DISEASE, vol. 4, no. 7, 1 juillet 2013 (2013-07-01) , page e719, XP055236407, DOI: 10.1038/cddis.2013.249 cité dans la demande
- KEEFE T. CHAN ET AL: "Intravital imaging of a spheroid-based orthotopic model of melanoma in the mouse ear skin", INTRAVITAL, vol. 2, no. 2, 1 avril 2013 (2013-04-01), page e25805, XP055236427, DOI: 10.4161/intv.25805
- MATHES STEPHANIE H ET AL: "The use of skin models in drug development", ADVANCED DRUG DELIVERY REVIEWS, vol. 69, 27 décembre 2013 (2013-12-27), pages 81-102, XP028648699, ISSN: 0169-409X, DOI: 10.1016/J.ADDR.2013.12.006

## Description

### DOMAINE DE L'INVENTION

La présente invention concerne un explant de peau contenant au moins un sphéroïde, son procédé d'obtention et un kit de mise en oeuvre de ce procédé, ainsi que ses utilisations pour l'identification de composés présentant un effet anti-cancéreux ou pour l'obtention d'échantillons pileux.

### ART ANTERIEUR

Les cellules cultivées en monocouche (2D) acquièrent un phénotype fondamentalement différent de celui des mêmes cellules *in vivo.* Ce phénomène provient d'une altération d'au moins une des fonctions liées à la prolifération, à la différenciation, à la survie, à l'architecture cellulaire ou encore à la migration cellulaire. Ces modifications induisent chez des cellules cultivées en 2D une sensibilité aux composés thérapeutiques distincte de celle observée chez les mêmes cellules *in vivo,* rendant ainsi l'identification de nouvelles molécules thérapeutiques particulièrement complexe et incertaine.

Pour pallier cet inconvénient, des modèles de culture cellulaire tridimensionnelle (3D) ont été mis au point dans le but de se rapprocher au plus près des conditions physiologiques rencontrées par les cellules dans l'organisme. Les méthodes 3D actuellement utilisées dérivent principalement de la technique de la goutte suspendue (Foti R, 2011, A simple hanging drop cell culture protocol for génération of 3D spheroid, J Vis Exp, 51 : pii 2720), dans laquelle la gravité entraîne une agrégation spontanée des cellules sur le ménisque de la goutte de milieu de culture qui les contient. D'autres techniques bien connues de l'Homme du métier consistent soit à mélanger une suspension cellulaire avec une matrice extracellulaire capable de se solidifier, soit à ensemencer une matrice de polymère avec un mélange cellule/matrice extracellulaire (Lee et al, 2007, Three-dimensional culture model of normal and malignant breast epithelial cells, Nat Methods, 4 : 359-365/Fischbach et al, 2007 Engineering tumors with 3D scaffolds, Nat Methods, 4 : 855-860). En dépit d'une relative hétérogénéité cellulaire qui résulte de la formation aléatoire de sphéroïdes, ces modèles sont couramment utilisés en cancérologie pour l'identification de composés d'intérêt thérapeutique. Toutefois, de tels modèles ne tiennent pas compte de l'environnement tissulaire existant, particulièrement pertinent dans le cadre de l'étude de cancers de la peau.

En 2013, un modèle 3D de mélanome métastatique a été mis au point sur une peau artificielle. Des sphéroïdes de la lignée cancéreuse 451-Lu ont été ajoutés à une coculture de kératinocytes/fibroblastes insérés dans une matrice de collagène de type I. Après 31 jours de culture, l'exposition de ce modèle de culture à des agents anti-cancéreux montre une réponse des cellules 451-Lu différentes de celle observée pour ces mêmes cellules dans un modèle de culture 2D. Néanmoins, l'auteur souligne l'absence des cellules du système immunitaire, des cellules endothéliales et d'autres types cellulaires normalement présentes dans le microenvironnement du mélanome *in vivo* (Vörsmann et al, 2013, Development of a human 3D organotypic skin-melanoma spheroid model for in vitro drug testing, Cell Death and Disease, 4, e719).

Des méthodes d'imagerie ont été développées pour suivre le développement tumoral dans un modèle murin d'implantation *in vivo* dans le derme de l'oreille de sphéroïde contenant des cellules de mélanome (Keefe et al., 2013, Intravital imaging of a spheroid-based orthotopic model of melanoma in the mouse ear skin, IntraVital, 2 :2, e25805). Néanmoins, cette approche ne permet d'observer les évènements cellulaires que le temps d'anesthésie des souris. En outre, les expérimentations animales sont de plus en plus ostracisées.

Il existe par conséquent un réel besoin de mettre au point un modèle plus proche de la peau naturelle.

Toutefois, la manipulation des sphéroïdes s'avère délicate pour les raisons suivantes :
- lors de l'injection d'une solution contenant un ou plusieurs sphéroïdes dans le derme de la peau à l'aide d'une seringue et d'une aiguille, ces derniers peuvent rester coincés dans l'aiguille sans être entraînés par la solution injectée. Une alternative à cela est d'utiliser des systèmes d'injection à déplacement positif où un piston pousse la solution lors de l'injection. La limite de ce système réside toutefois dans le risque d'altérer la structure de ces sphéroïdes, altérant ainsi les cellules qui le composent,
- lors de l'injection de solution, le derme étant élastique, il gonfle sous la pression de la solution injectée avant de se rétracter et ainsi créer un reflux de la solution, lequel peut entraîner le/les sphéroïde(s) hors du derme,
- lorsqu'on injecte une solution contenant un/des sphéroïdes dans le derme de la peau, il est difficile de contrôler, sous l'effet de la pression exercée par la seringue, la dispersion du/des sphéroïdes,
- lorsqu'on injecte une solution contenant des sphéroïdes dans le derme de la peau, il est nécessaire de mettre en oeuvre un volume conséquent pour entraîner les sphéroïdes hors de l'aiguille. Ceci peut provoquer une dilatation excessive du derme pouvant entraîner une réponse inflammatoire ou bien la rupture de la structure de la peau,
- dans le cas de l'injection de sphéroïdes en solution, il est relativement difficile de les aspirer dans l'aiguille et de les y garder sans qu'ils sédimentent d'un côté ou de l'autre de l'aiguille, rendant leur dispersion incontrôlable.

De manière surprenante, les inventeurs ont mis au point un procédé *in vitro* qui permet de pallier tous ces inconvénients. L'enrobage du sphéroïde dans une matrice biologiquement dégradable permet tout à la fois :
- de maintenir l'intégrité structurale du sphéroïde et de l'explant de peau qui l'accueille,
- de réduire le volume à injecter dans le derme de la peau,
- de contrôler le nombre de sphéroïdes injectés,
- de contrôler la dispersion/répartition des sphéroïdes au sein du derme,
- de visualiser précisément le site d'implantation du/des sphéroïdes.

Un tel procédé procure un gain de temps en terme de culture, une bonne reproductibilité et offre un choix de matrices biologiquement dégradables propice à chaque type cellulaire. Enfin, le procédé selon l'invention n'induit aucune interférence entre le sphéroïde et le tissu au sein duquel il est implanté, recréant ainsi un microenvironnement comparable à celui de la physiologie.

### RESUME DE L'INVENTION

La présente invention se rapporte à un procédé d'obtention d'explant de peau contenant au moins un sphéroïde. Les explants de peau utilisés pour cette invention sont issus de peau saine. Les sphéroïdes peuvent contenir des cellules cancéreuses/tumorales ou des cellules de follicules pileux, de glandes sébacées, de glandes sudoripares, de corpuscules sensoriels. Les explants de peaux contenant au moins un sphéroïde obtenus par le procédé de l'invention constituent des modèles tridimensionnels particulièrement intéressants pour identifier de nouveaux composés à visée thérapeutique.

Ainsi, un premier objet de l'invention concerne un procédé *in vitro* pour implanter des sphéroïdes dans un explant de peau, ledit procédé comprenant une étape d'implantation d'au moins un sphéroïde au sein dudit explant de peau. De préférence, ledit sphéroïde est enrobé dans une matrice biologiquement dégradable.

Le procédé en question peut viser à modéliser une tumeur de la peau, le ou les sphéroïdes implantés sont alors constitués de cellules tumorales.

Le procédé selon l'invention peut également viser à obtenir des échantillons pileux, le ou les sphéroïdes implantés sont alors constitués de cellules de follicules pileux.

Un autre objet de la présente invention se rapporte à un explant de peau contenant au moins un sphéroïde et obtenu par le procédé décrit précédemment.

Encore un autre objet de l'invention concerne l'utilisation d'un explant de peau contenant au moins un sphéroïde constitué de cellules tumorales pour l'identification de composés présentant un effet anti-cancéreux.

Encore un autre objet de l'invention concerne l'utilisation d'un explant de peau contenant au moins un sphéroïde constitué de cellules de follicules pileux pour l'obtention d'échantillon pileux.

Enfin, un autre objet de l'invention porte sur un kit permettant la mise en oeuvre du procédé de l'invention, ledit kit comprenant des cellules à même de former des sphéroïdes, une matrice biologiquement dégradable, un explant de peau et éventuellement un dispositif d'implantation.

### DESCRIPTION DES DESSINS

La figure 1 illustre le procédé de marquage/coloration d'un sphéroïde.
La figure 2 illustre le procédé d'inclusion d'un sphéroïde dans la matrice biologiquement dégradable.
La figure 3 illustre le procédé d'implantation d'un sphéroïde dans l'explant de peau.
La figure 4 montre l'implantation d'un sphéroïde dans un explant de peau après 24h de culture.
La figure 5 illustre le procédé d'implantation d'un sphéroïde en solution visqueuse.
La figure 6 montre l'implantation en gélatine 100 mg/mL d'un sphéroïde de la lignée WM-266-4, quelques minutes après son implantation dans un explant de peau (coloration Hématoxyline-Eosine).
La figure 7 montre l'implantation en agarose low melting 1% d'un sphéroïde de la lignée WM-266-4, 24 heures après son implantation dans un explant de peau (coloration Hématoxyline-Eosine).
La figure 8 montre l'implantation en PBS d'un sphéroïde de la lignée WM-266-4, 24 heures après son implantation dans un explant de peau (coloration Hématoxyline-Eosine).
La figure 9 montre l'implantation en acide hyaluronique 10 mg/mL d'un sphéroïde de la lignée WM-266-4, quelques minutes après son implantation dans un explant de peau. Le sphéroïde est détecté par fluorescence (délimitation du sphéroïde par le trait noir continu) et la structure de la peau est rendue visible en lumière transmise.
La figure 10 montre l'implantation en gélatine 100 mg/mL d'un sphéroïde de la lignée WM-266-4, quelques minutes après son implantation dans un explant de peau. Le sphéroïde est détecté par fluorescence (délimitation du sphéroïde par le trait noir continu) et la structure de la peau est rendue visible en lumière transmise.

### DESCRIPTION DETAILLEE DE L'INVENTION

L'invention a pour but de proposer un procédé *in vitro* reproductible et maitrisé d'implantation d'amas cellulaires dans le derme d'un explant de peau, lequel procédé offre un nouvel outil de culture organotypique convenant à l'étude de la biologie cutanée.

Un premier objet de l'invention concerne un procédé *in vitro* pour implanter des sphéroïdes, dans un explant de peau, ledit procédé comprenant une étape c) d'implantation, au sein dudit explant de peau, d'au moins un sphéroïde, enrobé de préférence dans une matrice biologiquement dégradable.

Par « amas cellulaires », on désigne un ensemble de cellules dont la taille varie de 10 à plusieurs milliers de cellules, de préférence une taille supérieure à 10³ cellules, et de préférence encore une taille supérieure à 10⁴ voir 10⁵ cellules. Les cellules qui forment cet amas peuvent avoir été préalablement dissociées ou non. Une telle dissociation est généralement obtenue par voie enzymatique selon des méthodes bien connues de l'Homme du métier. Les cellules qui composent l'amas cellulaire peuvent avoir des contacts et des interactions d'intensité variable qui conduisent à des organisations spatiales distinctes. Ainsi, un amas cellulaire résultant de la dissociation de cellules ne possède pas de structure spatiale organisée à T0, contrairement à un amas cellulaire constitué de cellules préalablement non dissociées. Maintenant, un amas cellulaire correspond littéralement à une concentration/accumulation locale de cellules qui forme donc une masse, même si les cellules ne sont pas associées et qui diffère donc de cellules en solution, lesquelles ne forment en aucun cas une masse. Toutefois, cette structure peut évoluer sous réserve que cet amas cellulaire soit mis en culture pendant plusieurs jours. Le sphéroïde constitue alors une organisation spatiale particulière d'amas cellulaire.

Par « sphéroïde », on désigne un ensemble de cellules organisées en multicouches concentriques et possédant une structure géométrique en forme de sphère. Les sphéroïdes se distinguent des agrégats de cellules qui constituent des structures cellulaires facilement dissociables (en raison de l'absence de liaisons cellules-cellules ou cellules-matrice). Par ailleurs, de tels agrégats sont dépourvus de structure sphérique. En lien avec la définition d'un sphéroïde, on pourra se reporter aux différentes revues sur le sujet (Kunz-Schuhghart et al, 1998, Multicellular spheroids : A three-dimensional in vitro culture system to study tumour biology, Int. J. Exp. Path. 79, 1-23 / Lin et al, 2008, Recent advances in three-dimensional multicellular spheroid culture for biomedical research, Biotechnol. J. 3, 1172-1184 / Hirschhaeuser et al, 2010, Multicellular tumor spheroids: an underestimated tool is catching up again, Journal of Biotechnology 148, 3-15).

Le diamètre des sphéroïdes utilisés dans le procédé selon la présente invention varie de 100 à 1000 µm, en particulier de 300 à 600 µm, et plus particulièrement ont un diamètre d'au moins 500 µm.

Par « implantation », on entend l'introduction d'un sphéroïde, dans le derme d'un explant de peau. Cette introduction se caractérise par une insertion du sphéroïde directement dans la structure du derme. L'introduction du sphéroïde dans le derme s'effectue à l'aide d'un dispositif d'implantation, notamment une aiguille creuse, biseautée ou non, ou de tout autre dispositif mécanique équivalent connu de l'Homme du métier. Dans les heures qui suivent l'insertion du sphéroïde dans le derme, ce site d'insertion devient un site d'ancrage pour le sphéroïde dans le derme.

Par « derme », on désigne le tissu sous-jacent de l'épiderme qui contient des réseaux vasculaires et nerveux denses ainsi que des annexes kératinisées prolongeant l'épiderme, ce tissu inclut également les follicules pileux, les glandes sébacées et les glandes sudoripares.

Le derme est subdivisé en deux parties qui diffèrent par la composition et l'organisation de leur matrice extracellulaire respective. Le derme papillaire se trouve directement sous l'épiderme. Il s'agit d'un tissu conjonctif lâche constitué de fines fibrilles de collagène de type I et III et des fibres élastiques qui sont orientées perpendiculairement à l'épiderme formant ainsi des structures en forme de chandelles. Le derme réticulaire, placée sous le derme papillaire, est également un tissu conjonctif composé d'un entrecroisement de faisceaux de grosses fibres de collagène et de fibres élastiques qui présentent une orientation préférentiellement parallèle à la surface de la peau. Le derme réticulaire contient moins de collagène de type III que le derme papillaire.

Par « explant de peau », on désigne un fragment ou une biopsie de peau qui comprend au moins l'épiderme, le derme et les annexes épidermiques. Ce fragment ou biopsie de peau pourra comprendre également une partie du tissu sous-cutané encore appelé hypoderme.

De préférence, ledit fragment ou biopsie de peau est prélevé chez un mammifère, en particulier chez l'humain ou chez le porc. Dans le but d'assurer sa survie *in vitro,* le prélèvement doit avoir été réalisé dans un délai de 1h à moins de 72h, de préférence de 1h à moins de 48h avant l'implantation de l'amas cellulaire, en particulier du sphéroïde. Les techniques de prélèvement d'explant de peau sont bien connues de l'Homme du métier.

Selon un mode de réalisation particulier, le procédé *in vitro* selon l'invention comprend, préalablement à l'étape c), une étape b) d'enrobage dudit au moins un sphéroïde dans une matrice biologiquement dégradable.

Par « matrice biologiquement dégradable », on désigne un ensemble de molécules à même de former une enveloppe autour d'un sphéroïde, notamment par polymérisation, et naturellement dégradées par les constituants du derme. Cette dégradation intervient dans les heures qui suivent l'implantation du sphéroïde dans l'explant de peau, permettant ainsi l'interaction directe du sphéroïde avec l'explant de peau. La matrice biologiquement dégradable a pour but de permettre l'implantation du sphéroïde dans l'explant de peau mais doit disparaitre rapidement pour ne pas interférer dans les interactions explant de peau / cellules implantées. Parmi les matrices biologiquement dégradables envisageables, l'Homme du métier au regard de ses connaissances générales choisira celle la plus adaptée au type cellulaire formant le sphéroïde.

Selon un mode de réalisation particulier, la matrice biologiquement dégradable est choisie dans le groupe comprenant ou consistant en la gélatine, les collagènes (en particulier les collagènes de type I, III, IV ou V), les glycosaminoglycanes et les protéoglycanes de la matrice extracellulaire (acide hyaluronique), l'élastine, les polysaccharides (agarose), une matrice synthétique biocompatible ou leur mélange.

Par «enrobage », on désigne l'action qui consiste à inclure/introduire/insérer le sphéroïde dans la matrice biologiquement dégradable, laquelle se trouve de préférence en légère surfusion. La température de la matrice biologiquement dégradable doit permettre le maintien en vie des cellules du sphéroïde et ainsi ne doit pas dépasser 37°C au moment de l'enrobage. Par ailleurs, la matrice biologiquement dégradable doit posséder une température de solidification/polymérisation à température ambiante (i.e. de 15°C à 25°C).

Dans un mode de réalisation spécifique, la matrice biologiquement dégradable sera la gélatine. L'origine de cette gélatine est animale, en particulier de boeuf ou de porc.

Avantageusement on utilisera une gélatine sous la forme d'une solution aqueuse dont la concentration en gélatine varie de 80 à 300 mg/ml, par exemple de 120 à 300 mg/ml, de préférence de 100 à 250 mg/ml, et de préférence encore de 100 à 150 mg/ml.

Dans un autre mode de réalisation spécifique, la matrice biologiquement dégradable est un glycosaminoglycane, de préférence de l'acide hyaluronique.

Avantageusement, cet acide hyaluronique sera sous la forme d'une solution aqueuse dont la concentration (en acide hyaluronique) est comprise entre 2 et 30 mg/ml, préférentiellement entre 3 et 20 mg/ml, en particulier entre 5 et 10 mg/ml.

Dans encore un autre mode de réalisation spécifique, la matrice biologiquement dégradable est un polysaccharide, de préférence de l'agarose et, de préférence encore, de l'agarose à faible point de fusion ou « low melting ».

Plus spécifiquement encore, on utilisera l'agarose « low melting » sous la forme d'une solution aqueuse dont la concentration en agarose « low melting » est comprise entre 0,5 à 5% (en poids), préférentiellement entre 0,8 et 2%, et en particulier entre 1 et 1,5%.

Il est préférable que la viscosité de la matrice biologiquement dégradable soit telle, après polymérisation, qu'elle permette de ne pas altérer la structure du sphéroïde, tout en conférant une fermeté suffisante pour permettre son implantation dans l'explant, notamment par son expulsion de l'aiguille.

Avantageusement, la matrice biologiquement dégradable présente, après l'étape d'enrobage du sphéroïde dans la matrice biologiquement dégradable, une viscosité comprise entre 0,5 et 10⁴ Pa.s, de préférence entre 1 et 10³ Pa.s de sorte que l'enrobé soit sous une forme visqueuse ou sous la forme d'un gel.

Selon un mode de réalisation particulier, le sphéroïde,est implanté en solution visqueuse (de 1 à 50 Pa.s) dans l'explant de peau.

Selon un mode de réalisation particulier, le procédé de l'invention comprend, en préalable à l'étape c) et à l'étape b), une étape a) de coloration dudit au moins un sphéroïde.

Par « coloration », on désigne une étape de marquage du sphéroïde, qui vise à le rendre apparent en lumière visible ou invisible en utilisant une sonde. Le but de cette étape de coloration est de pouvoir visualiser le ou les phéroïdes à implanter. Ceci est notamment réalisable par inclusion de pigments provenant de colorants naturels ou artificiels (cas des sondes colorantes). Il est également envisageable de colorer le sphéroïde avec une sonde fluorescente, notamment un fluorophore ou un fluorochrome. Dans ce cas, la détection du sphéroïde est opérée sous émission de fluorescence.

En particulier, l'étape de coloration utilise une sonde colorante choisie dans le groupe comprenant ou consistant en le bleu de méthylène, le vert méthyle, le vert malachite, l'amido black, le cristal violet, le rouge neutre ou le bleu de Coomassie.

Encore plus particulièrement, l'étape de coloration utilise une sonde fluorescente choisie dans le groupe comprenant ou consistant en les carbocyanines lipophiliques Dil (DilC18(3)), DiO (DiOC18(3)), DiD (DilC18(5)) et DiR (DilC18(7)), le NUCLEAR-ID^{™}, le CELL-MASK^{™} ou le CELL-TRACKER^{™}, ou des nanoparticules (Quantum dots ou nanoparticules d'or).

Cette étape de coloration peut également être réalisée avec tout autre type de sonde, de préférence une sonde biocompatible, c'est-à-dire une sonde qui n'induise pas de mort cellulaire au sein du sphéroïde.

Selon un mode de réalisation particulier, le procédé selon l'invention comprend en outre une étape d) de mise en culture de l'explant de peau après implantation dudit au moins un sphéroïde à l'issu de l'étape c), de préférence jusqu'à dégradation de la matrice biologiquement dégradable.

Une telle étape de mise en culture peut être réalisée selon les techniques classiques de culture d'explants de peau bien connues de l'Homme du métier. A titre d'exemple, de telles méthodes sont explicitées dans la demande internationale WO 2013/164436.

Par « mise en culture », on désigne la maintenance de l'état physiologique et morphologique de l'explant de peau et du sphéroïde qui y est implanté, c'est-à-dire de l'intégralité des cellules qui les composent. La durée de mise en culture varie en fonction de la nature de la matrice biologiquement dégradable, et en particulier de la vitesse à laquelle elle est dégradée par les enzymes situées dans le derme de l'explant de peau.

Par « dégradation de la matrice biologiquement dégradable », on désigne la digestion de tout ou partie de la matrice biologiquement dégradable par les enzymes présentes dans le derme de l'explant de peau. Ces enzymes sont notamment des collagénases synthétisées par les fibroblastes du derme ou des protéases contenues dans les granules des mastocytes du derme. La digestion de la matrice biologiquement dégradable conduit à l'obtention de peptides ou d'acides aminés qui sont rapidement éliminés.

De préférence, la dégradation de la matrice biologiquement dégradable permet un contact du sphéroïde, et de ses cellules avec le derme. Avantageusement, cette dégradation est totale.

Selon un autre mode de réalisation, la dégradation de la matrice biologiquement dégradable peut être partielle en ce sens qu'il subsiste une fine couche de matrice biologiquement dégradable entre le sphéroïde et les cellules du derme. Cette interface matricielle permet toutefois une diffusion correcte des métabolites et des composés entre le derme et le sphéroïde.

La durée de mise en culture de l'explant pourra également varier en fonction de la nature des cellules du sphéroïde. Ainsi, dans le cas de sphéroïdes comprenant des cellules à forte capacité multiplicative, la durée de culture de l'explant de peau ne devra pas excéder celle du grossissement du sphéroïde qui pourrait altérer l'intégrité tissulaire de l'explant. Inversement, dans le cas de cellules à croissance lente ou pour lesquelles une différenciation est nécessaire, la durée de culture de l'explant de peau pourra dépasser plusieurs jours.

Dans le procédé selon l'invention, ledit explant de peau est une biopsie de peau saine.

La biopsie peut provenir d'une plastie de n'importe quelle partie du corps d'un mammifère, de préférence d'un humain, comme par exemple de l'abdomen, de la poitrine, des fesses, du dos, du cuir chevelu, etc.

De préférence, la biopsie de peau est de forme cylindrique.

Toutefois, toute autre forme géométrique de biopsie de peau convient également au procédé selon l'invention, en particulier une forme carrée, rectangulaire, ovale, triangulaire, etc....

De préférence, la forme cylindrique possède un diamètre qui varie de 1 mm à 50 mm, plus particulièrement de 5 mm à 20 mm, encore plus particulièrement de 7 mm à 17 mm, et une épaisseur qui varie de 1 mm à 20 mm, plus particulièrement de 2 mm à 15 mm, encore plus particulièrement de 2 mm à 10 mm, et encore plus particulièrement de 2 mm à 5 mm.

Selon un mode de réalisation particulier, ledit au moins un sphéroïde utilisé dans le procédé selon l'invention est constitué de cellules tumorales et il vise à modéliser une tumeur de la peau.

Par « cellules tumorales », on désigne des cellules possédant un dysfonctionnement au niveau du cycle cellulaire qui entraine une hyperprolifération cellulaire. Ce dysfonctionnement est généralement dû à une altération génétique somatique d'un oncogène, d'un gène suppresseur de tumeur ou d'un gène impliqué dans la réparation de l'ADN.

Par « tumeur de la peau », on désigne une masse constituée de cellules cancéreuses, également et indifféremment appelées cellules tumorales. La gravité des tumeurs de la peau réside dans le fait que pour la plupart d'entre elles, les cellules qui les forment peuvent se propager vers d'autres parties du corps pour y former des métastases. Les tumeurs malignes de la peau se répartissent en deux familles principales : les carcinomes et les mélanomes.

Les carcinomes cutanés se développent en général sur les parties du corps souvent exposées au soleil comme le visage, le cou, les épaules, les avant-bras ou le dos des mains. Ils surviennent en général après 50 ans et évoluent lentement. Ils sont très fréquents, mais facilement guérissables dans la majorité des cas. Les principaux types de carcinomes sont les carcinomes basocellulaires et les carcinomes épidermoïdes cutanés (également appelés carcinomes spinocellulaires).

Le mélanome cutané représente une minorité des cancers de la peau mais constitue le plus grave d'entre eux. Il survient à tout âge chez les adultes et est très rare chez l'enfant. Le mélanome apparaît sur n'importe quelle partie du corps, le plus souvent sur le tronc chez l'homme et sur les jambes chez la femme. Son apparition est également liée à l'exposition au soleil. Il peut évoluer rapidement et se propager. Le mélanome se manifeste soit par l'apparition d'une tâche pigmentée sur la peau saine qui ressemble à un naevus, soit par la modification de couleur et de forme d'un naevus préexistant. Les principaux types de mélanomes de la peau sont : le mélanome superficiel extensif, le mélanome nodulaire, le mélanome de Dubreuilh et le mélanome acro-lentigineux.

Selon un mode de réalisation plus particulier, les cellules tumorales constituant le sphéroïde utilisé dans le procédé selon l'invention sont des cellules de mélanomes ou de carcinomes (e.g. baso- ou spinocellulaire).

Selon un autre mode de réalisation, les cellules constitutives du sphéroïde utilisé dans le procédé de l'invention sont issues d'une pathologie cutanée autre qu'un cancer, en particulier ces cellules sont issues de pathologies inflammatoires de la peau telles qu'une plaque de psoriasis, une lésion d'eczéma, ou une dermatite atopique. En particulier, dans le cadre de pathologies inflammatoires cutanées, les cellules constitutives du sphéroïde sont des cellules de types fibroblastiques, des kératynocytes, des cellules endothéliales ou des cellules nerveuses.

Selon un mode de réalisation particulier, le procédé de l'invention vise à identifier des composés présentant un effet anti-cancéreux et il comprend en outre les étapes de :
e) Mise en contact d'au moins un composé avec un explant de peau à l'issue de l'étape d),
f) Mise en culture du mélange issu de l'étape e), et
g) Identification des composés présentant un effet anti-cancéreux à l'issue de l'étape f).

Par « effet anti-cancéreux », on désigne la capacité d'un composé à interagir avec le cycle cellulaire des cellules tumorales du sphéroïde. La mesure du taux de survie ou du niveau de prolifération des cellules tumorales du sphéroïde implanté dans l'explant de peau constitue un bon indicateur de l'effet anti-cancéreux d'un composé. L'effet anti-cancéreux peut être évalué relativement à un composé contrôle ou témoin mis en contact avec un explant de peau tel qu'obtenu par le procédé *in vitro* selon l'invention et ne possédant pas d'effet anti-cancéreux, ou relativement à un explant de peau tel qu'obtenu par le procédé selon l'invention qui n'est mis au contact d'aucun composé. Les techniques de mesure d'apoptose, du taux de nécrose ou la détermination des différentes phases du cycle cellulaire sont bien connues de l'Homme du métier.

De préférence, à l'issue de l'étape f) du procédé selon l'invention, on procédera à la sélection des composés qui sont associés à un taux de cellules mortes ou ne proliférant plus au sein de l'amas cellulaire d'au moins 10%, particulièrement d'au moins 30%, plus particulièrement d'au moins 50%, encore plus particulièrement d'au moins 90%.

Avantageusement, les composés sélectionnés sont ceux n'induisant pas ou peu de mort cellulaire parmi les cellules de l'explant de peau, autre que celles du sphéroïde. Par « n'induisant pas de mort cellulaire », on entend un taux de mort cellulaire qui soit au maximum supérieur à 10% à celui observé en l'absence du composé, de préférence au maximum supérieur à 5% et, de manière particulièrement préféré au maximum supérieur à 2%.

Par « composé », on désigne des composés chimiques ou des vecteurs d'expression qui peuvent provenir de banques, notamment à visée de criblage, ou de composés nouvellement synthétisés. Des vecteurs d'expression contenant un acide nucléique d'intérêt pourront notamment être utilisés dans la perspective de modifier le niveau d'expression d'un gène cible des cellules du sphéroïde.

Les composés identifiés par le procédé de l'invention peuvent, dans une étape ultérieure, être testés sur des modèles animaux de pathologies cutanées, ceci afin de vérifier leur efficacité *in vivo.*

Un avantage des explants de l'invention est de pouvoir analyser simultanément l'effet des composés sur les cellules du sphéroïde et sur les cellules du derme et de l'épiderme de l'explant de peau. Ceci permet de discriminer les composés possédant à la fois un effet anti-cancéreux sur les cellules tumorales du sphéroïde et un effet toxique sur les cellules du derme et de l'épiderme, des composés possédant un effet anti-cancéreux sur les cellules tumorales du sphéroïde et qui sont non toxiques pour les cellules du derme et de l'épiderme. En d'autres termes, la mesure de l'efficacité des composés vis-à-vis des cellules tumorales (sphéroïde) et la mesure de leur innocuité vis-à-vis des cellules saines (explant de peau) peuvent être réalisées au cours d'une même expérience, de manière synchrone. Ainsi, l'identification des composés anti-cancéreux et non toxiques est réalisée en un seul criblage, engendrant ainsi un gain de temps considérable et une réduction des coûts.

Selon un autre mode de réalisation particulier, ledit au moins un sphéroïde utilisé dans le procédé de l'invention est constitué de cellules de follicule pileux, de glandes sébacées, de glandes sudoripares ou de corpuscules sensoriels et il vise à l'obtention d'échantillons pileux.

Par « obtention d'échantillons pileux », on désigne l'organisation de cellules en plusieurs couches distinctes et identifiables de sorte à recréer une structure similaire à celle d'un follicule pileux naturel. Ainsi, les échantillons pileux obtenus selon le procédé *in vitro* de l'invention possèdent une densité de follicules pileux pouvant varier de 1 à plusieurs dizaines par explant de peau.

Selon un autre mode de réalisation plus particulier, les cellules de follicules pileux constituant le sphéroïde utilisé dans le procédé selon l'invention sont choisies dans le groupe comprenant ou consistant en les cellules dermiques de la papille, les cellules progénitrices de la matrice, les cellules souches du bulbe ou leur mélange.

Ces différents types cellulaires sont à l'origine de la morphogénèse *in vitro* de nouveaux follicules pileux. Tout comme les follicules pileux naturels, leur structure comprend une papille dermique, une gaine épithéliale interne et externe, un muscle horripilateur, une glande sébacée et une tige pilaire.

Un autre objet de l'invention concerne un explant de peau contenant au moins un sphéroïde obtenu par le procédé décrit précédemment.

Un tel explant de peau est qualifié de « en culture » et se trouve par exemple dans une boîte, plaque ou flasque de culture et en présence de milieu de culture.

Plus particulièrement, ledit au moins un sphéroïde implanté dans l'explant de peau est constitué de cellules tumorales.

Encore plus particulièrement, ledit au moins un sphéroïde implanté dans l'explant de peau est constitué de cellules de follicules pileux.

Encore un autre objet de l'invention concerne l'utilisation d'un explant de peau contenant au moins un sphéroïde obtenu par le procédé décrit précédemment pour l'identification des composés présentant un effet anti-cancéreux.

Les composés identifiés selon cette utilisation de l'invention peuvent servir de base à l'élaboration de traitements thérapeutiques pour un patient souffrant de cancer cutané. Une telle utilisation selon l'invention permet d'envisager une thérapie personnalisable d'un patient à l'autre en fonction de l'intensité de l'effet anti-cancéreux des différents composés testés sur les cellules des sphéroïdes.

Selon un autre mode de réalisation, un explant de peau contenant au moins un sphéroïde obtenu par le procédé selon l'invention pourra être utilisé pour l'obtention d'échantillons pileux.

De tels échantillons pileux peuvent notamment être réimplantés sur le cuir chevelu de patients atteints d'alopécie ou qui ont subit un traitement radio- ou chimio-thérapeutique.

Encore un autre objet de l'invention concerne un kit de mise en oeuvre du procédé *in vitro* selon l'invention, ledit kit comprenant des cellules à même de former un sphéroïde, une matrice biologiquement dégradable, un explant de peau, et, éventuellement, un dispositif d'implantation d'un sphéroïde.

Dans un kit selon l'invention, les cellules à même de former un sphéroïde sont des cellules tumorales ou des cellules issues de follicules pileux.

Dans un kit selon l'invention, la matrice biologiquement dégradable est choisie dans le groupe comprenant ou consistant en la gélatine, le collagène de type I, III, IV ou V, les glycosaminoglycanes et les protéoglycanes de la matrice extracellulaire, l'élastine, les polysaccharides, une matrice synthétique biocompatible ou leur mélange.

Dans un kit selon l'invention, un explant de peau est une biopsie de peau saine.

Dans un kit selon l'invention, le dispositif d'implantation est une aiguille de format 14G à 29G, en particulier de 17G à 25G, plus particulièrement de 16G à 23G, montée sur une seringue, un capillaire, une tige creuse, un trocart ou tout autre dispositif mécanique susceptible de remplir les mêmes fonction qu'une aiguille à savoir le passage d'une matrice biologiquement dégradable et d'un sphéroïde, la polymérisation de ladite matrice biologiquement dégradable autour du sphéroïde puis leur éjection dans un explant de peau. Le dispositif d'implantation pourra être pourvu ou non d'une extrémité biseautée dans le but de faciliter l'implantation du sphéroïde dans l'explant de peau.

Les exemples suivants sont donnés uniquement à titre d'illustration de l'objet de la présente invention dont ils ne constituent en aucune manière une limitation.

### EXEMPLES

### 1 - Préparation du sphéroïde

### 1.1 - Culture de sphéroïdes

Les sphéroïdes de cellules adhérentes sont obtenus par culture sur un support empêchant l'adhérence des cellules. Les interactions cellules-cellules étant ainsi favorisées, les cellules s'agrègent et forment une structure 3D solide. La technique de culture en plaque 96 puits est décrite ci-après.

La surface des puits de plaques de culture 96 puits est traitée avec un hydrogel n'ayant pas d'affinités avec les cellules (ex : Poly-HEMA) au moins 24h à l'avance. Les cellules d'une culture monocouche sont décollées de leur support à l'aide de trypsine afin d'obtenir une suspension cellulaire. Les cellules sont ensuite comptées pour déterminer la concentration cellulaire de la suspension. 100 µL de suspension cellulaire contenant de 500 à 5000 cellules sont ensemencées par puits de plaques 96 puits pré-traitées avec l'hydrogel précité. Les plaques 96 puits sont centrifugées 6 min à 190 g. Le rassemblement des cellules au centre des puits est vérifié sous microscope optique et les plaques 96 puits contenant les cellules sont incubées à 37°C, en présence de 5% de CO₂ sous atmosphère humide.

Les sphéroïdes se forment et sont manipulables après 24 à 48h selon les cellules testées. Leur milieu de culture est renouvelé tous les trois jours et leur diamètre est mesuré sous un microscope inversé jusqu'à ce qu'ils atteignent le diamètre souhaité.

Par exemple, les sphéroïdes HCT116 ensemencés à 2000 cellules à jour J0 atteignent un diamètre d'environ 550 µm à J+3.

Les sphéroïdes peuvent être produits à partir de lignées commerciales où à partir de cellules primaires issues de tissus normaux ou pathologiques issus d'exérèses : cancers cutanés (mélanome, cancers baso- ou spinocellulaires, autre type de cancers cutanés) plaques de psoriasis, lésion d'eczéma ou de dermatite atopique, ou tout autre type de pathologie cutanée.

### 1.2 - Marquage / coloration du sphéroïde

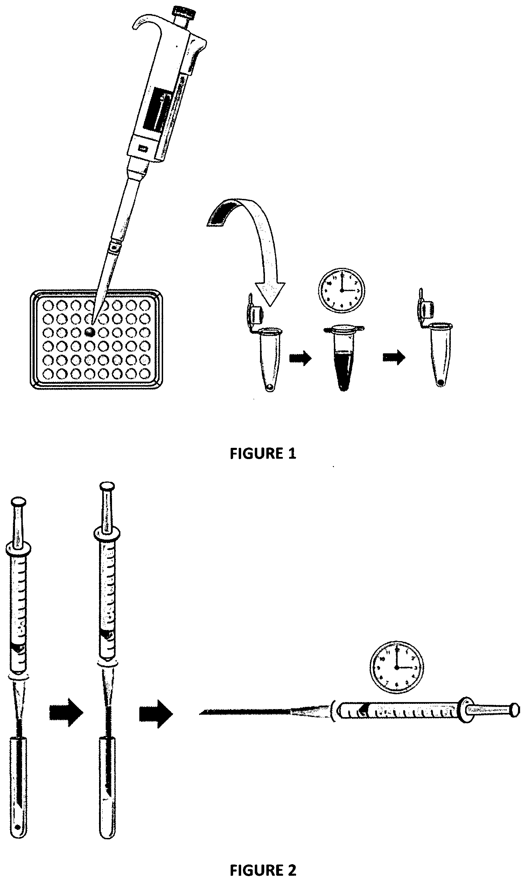

Le sphéroïde est récupéré de son puits de la plaque 96 puits et transféré dans un tube à fond conique de 1,5 ml de type EPPENDORF (figure 1). Puis le sphéroïde est marqué avec la sonde fluorescente biocompatible souhaitée selon le mode opératoire qui lui est propre. Par exemple, dans le cas du NUCLEAR-ID^{™}, le sphéroïde est incubé pendant 24h à 37°C dans une solution de NUCLEAR-ID^{™} à 1 µM dans du milieu de culture.

Le sphéroïde est coloré avec un colorant choisi dans le groupe comprenant ou consistant en le bleu de méthylène, le vert méthyle, le vert malachite, l'amido black, le cristal violet, le rouge neutre ou le bleu de Coomasie:
- le milieu de culture du sphéroïde est remplacé par la solution de colorant à la concentration préconisée et incubé le temps nécessaire à 37°C. Par exemple, dans le cas du vert malachite, le sphéroïde est incubé pendant 15 min à 37°C dans une solution de vert malachite à 10 mg/mL dans du PBS.
- le colorant est éliminé et rincé par bains successifs de PBS autant de fois que nécessaire pour éliminer tout le surplus de colorant.

### 1.3 - Inclusion dans la gélatine

Une solution de gélatine à 150 mg/mL est préparée et chauffée pour la rendre liquide au moins 1h à l'avance, puis sa température est rééquilibrée à 37°C. 1 mL de la solution de gélatine est prélevé et transféré dans un tube EPPENDORF. Le sphéroïde est pipeté dans un volume minimal de PBS et re-suspendu dans la solution de gélatine.

Une aiguille de jauge 16 à 23, plus particulièrement de 16 à 20 et encore plus particulièrement de 18 est montée sur une seringue de 0,5 à 10 mL et plus particulièrement de 0,5 à 5 mL, et encore plus particulièrement de 1 mL. A l'aide de la seringue, la gélatine, caractérisée par une viscosité à 35°C comprise entre 15 et 250 mPa.s est aspirée jusqu'à remplir la totalité du volume de l'aiguille (la gélatine doit commencer à remonter dans le volume mort situé entre l'aiguille et la seringue). Une fois l'aiguille remplie, aspirer le sphéroïde suspendu dans la gélatine afin que celui-ci reste dans les derniers microlitres de l'aiguille. Poser le dispositif seringue / aiguille contenant le sphéroïde à l'horizontale sous le PSM (Poste de Sécurité Microbiologique) et laisser prendre en masse la gélatine à température ambiante pendant minimum 10 minutes (figure 2).

### 2 - Implantation du sphéroïde

L'aiguille est détachée de la seringue. A l'aide d'une pince, l'explant de peau est maintenu à plat, derme sur le dessus. L'aiguille est introduite dans l'explant par le côté du derme, jusqu'à ce que le biseau soit entièrement rentré dans le derme. Le biseau de l'aiguille est placé au plus près de l'épiderme.

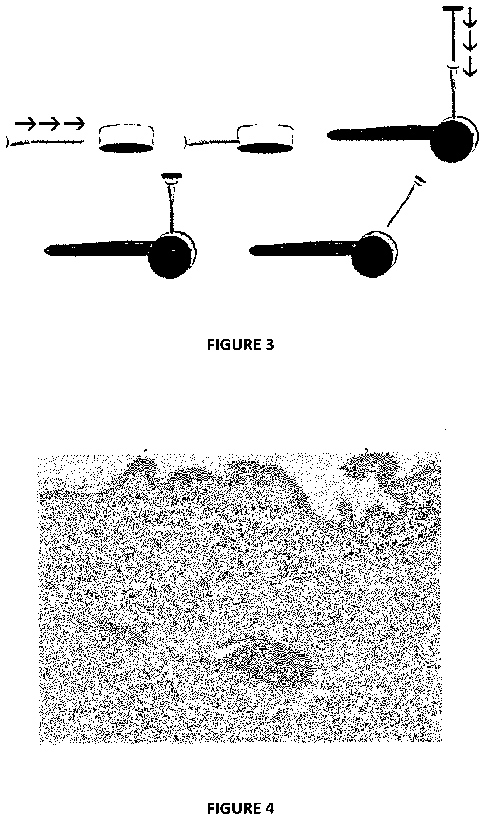

Tout en maintenant l'extrémité de l'aiguille dans l'explant de peau à l'aide d'une pince, un objet cylindrique stérilisé et dont le diamètre est inférieur au diamètre interne de l'aiguille est introduit dans l'aiguille. La gélatine contenant le sphéroïde est éjectée de l'aiguille en poussant l'objet cylindrique stérilisé à l'intérieur de l'aiguille jusqu'à la vider de tout son contenu. L'aiguille est retirée délicatement de l'explant de peau (figure 3).

### 3 - Culture ex vivo

L'explant implanté de son sphéroïde est monté sur une matrice liquide coulée dans un insert de culture selon le même procédé que celui utilisé pour le modèle NATIVESKIN^{™}, basé sur l'utilisation d'une matrice naturelle servant de support physique et de couche nourricière à des explants de peau ou d'épiderme humains cultivés in vitro. L'explant est ensuite cultivé en plaque (culture en incubateur, renouvellement quotidien du milieu de culture).

L'observation du sphéroïde enrobé a révélé que la gélatine utilisée lors de l'implantation est naturellement et rapidement dégradée par les constituants du derme dans les heures suivant son injection, permettant de ce fait l'interaction directe du sphéroïde avec l'explant de peau ce qui fait de celui-ci un modèle reproductible et appréciable.

### 4 - Analyse par histologie

### 4.1 - Coupe sur microtome

L'analyse peut se faire sur des échantillons inclus en bloc de paraffine par coupes sériées au microtome.

L'explant de peau dans lequel au moins un sphéroïde est implanté est déshydraté par un bain d'alcool puis passage dans un bain de xylène. Un premier bain dans la paraffine permet de remplacer l'eau préalablement contenue dans l'explant de peau par la paraffine.

Les échantillons imprégnés de paraffine sont sortis de leur bain et transférés dans un récipient dont le fond est tapissé de papier absorbant, afin d'être apportés à proximité de la station d'inclusion.

Les échantillons, enfermés dans des cassettes d'histologie, sont plongés dans la paraffine liquide à 56°C pour faire refondre la paraffine qui les imprègne.

Pour chaque échantillon : la cassette d'histologie est ouverte, l'échantillon est éventuellement coupé en deux. Un moule d'inclusion est rempli de paraffine liquide, et l'échantillon (ou les 2 morceaux d'échantillon) est placé dans le moule et orienté dans le sens désiré pour la coupe. Le moule est en même temps transféré sur un support réfrigéré afin de solidifier la paraffine du fond du moule et d'y maintenir l'échantillon. Le couvercle de la cassette d'histologie sur lequel figure la référence de l'échantillon est placé par-dessus, de telle façon que la paraffine le traverse (possibilité d'ajouter de la paraffine si besoin), puis le tout est placé au froid (réfrigérateur, freezer, chambre froide...) plusieurs minutes (5-6), afin de solidifier la paraffine en bloc, emprisonnant l'échantillon dans la bonne orientation et le couvercle de la cassette d'histologie qui deviendra le support du bloc.

Une fois le bloc bien solide, celui-ci est démoulé. L'excès de paraffine est éventuellement gratté à l'aide d'une spatule sur les côtés du couvercle de la cassette d'inclusion.

Des coupes sériées d'épaisseur variant de 4 à 5 µm sont alors réalisées sur toute la longueur du bloc de paraffine contenant l'échantillon.

La figure 4 illustre les résultats d'une telle coloration réalisée sur un explant de peau implanté avec un sphéroïde après 1 jour de culture. L'analyse du modèle par histologie (ex : coloration Hématoxyline et Eosine) permet d'attester de la présence d'un sphéroïde dans le derme de la biopsie de peau, d'observer sa structure et de suivre son évolution au cours de la culture.

### 4.2 - Cryosection

L'analyse peut également se faire sur coupe congelée afin de ne pas altérer la fluorescence des cellules / du(es) sphéroïde(s) à la déshydratation.

La culture de l'explant de peau dans lequel au moins un sphéroïde est implanté est arrêtée en fixant l'échantillon 24h dans 10 fois son volume de formaline 10%. L'explant est ensuite rincé dans 10 fois son volume de PBS, épongé sur un papier absorbant et transféré dans un tube EPPENDORF. Le tube EPPENDORF est plongé dans l'azote liquide jusqu'à la congélation de l'explant.

Dans l'enceinte du cryostat, l'explant congelé est inclus en OCT puis des tranches d'épaisseur constante de 3 à 15 µm de la partie de l'explant contenant le/le(s) sphéroïde(s) implanté(s) sont réalisées. Les tranches sont déposées sur lames qui sont séchées pendant quelques heures au minimum.

Une coloration classique à l'Hématoxyline et Eosine est réalisée.

### 5- Implantation du sphéroïde en solution visqueuse

### 5.1 - Composition des solutions

| **N°** | **Solution** |
|---|---|
| 1 | Phosphate Buffered Saline (PBS) |
| 2 | Gélatine : concentration variant de 100 mg/mL à 250 mg/mL |
| 3 | Acide hyaluronique : de 5 à 10 mg/mL |
| 4 | Agarose Low melting 1% |

### 5.2 - Implantation du sphéroïde en PBS

Les sphéroïdes issus des deux lignées cellulaires HCT116 et WM-266-4 ont été cultivés et colorés comme décrit aux points 1.1 et 1.2.

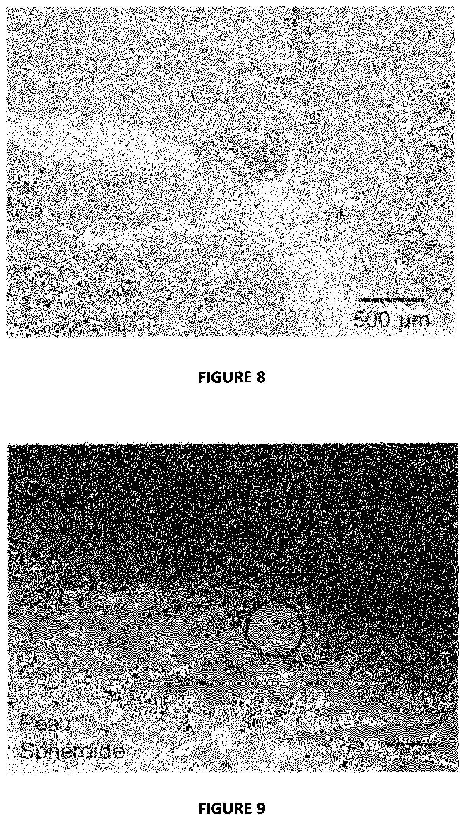

Le sphéroïde est transféré dans un tube contenant du PBS. Il est aspiré dans environ 10 µL de PBS dans une aiguille 18G montée sur une seringue 1 mL. Il est ensuite injecté comme précédemment décrit par le côté du derme d'un explant. La figure 8 illustre l'implantation d'un sphéroïde en PBS.

Cette méthode, très traditionnelle, ne permet pas d'implanter de façon systématique un sphéroïde dans un explant, elle a été testée à titre de comparaison avec les autres méthodes d'implantation d'un sphéroïde en matrice biodégradable.

### 5.3 - Implantation du sphéroïde en solution visqueuse

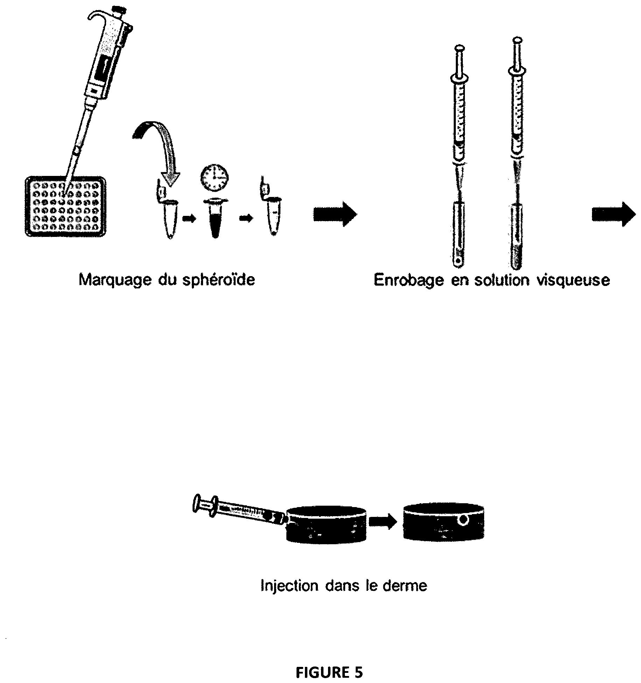

La figure 5 résume les différentes étapes d'implantation d'un sphéroïde en solution visqueuse. Les sphéroïdes issus des deux lignées cellulaires HCT116 et WM-266-4 ont été cultivés et colorés comme décrit au points 1.1 et 1.2.
- Inclusion dans la solution visqueuse :
   La solution de gélatine ou d'acide hyaluronique à la concentration donnée est préchauffée à 37°C dans un bain-marie à sec.

Le sphéroïde est prélevé de son puits de culture et transféré dans un tube EPPENDORF.

Son milieu de culture est aspiré et remplacé par 1 mL de solution visqueuse. Le tube est conservé à 37°C jusqu'à implantation.
- Implantation :
   Une aiguille de jauge 18 est montée sur une seringue de 1 mL.

10 µL de solution visqueuse sont aspirés dans l'aiguille, puis le sphéroïde est aspiré dans quelques µL de solution visqueuse supplémentaire.

L'injection du contenu de l'aiguille se fait immédiatement après enrobage afin que la solution reste visqueuse.

L'aiguille est introduite dans l'explant de peau (maintenu à plat à l'aide d'une pince, derme sur le dessus) par le côté du derme, jusqu'à ce que le biseau soit entièrement rentré dans le derme (comme précédemment).

Tout en maintenant l'extrémité de l'aiguille dans l'explant, la solution contenant le sphéroïde est éjectée de l'aiguille en utilisant la seringue à laquelle elle est reliée.

L'aiguille est ensuite délicatement retirée de l'explant de peau.

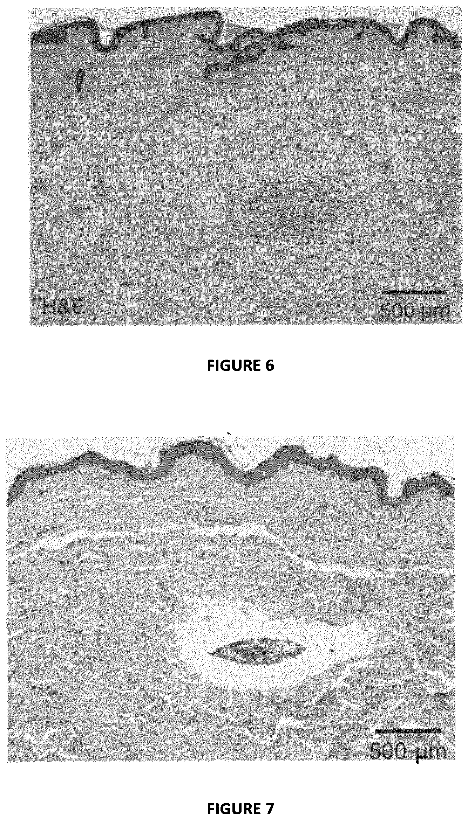

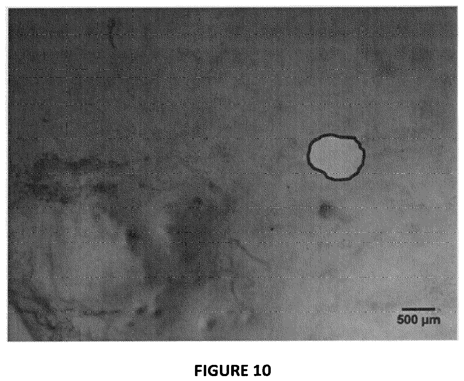

Les figures 6 et 10 illustrent l'implantation d'un sphéroïde en gélatine 100 mg/ml dans un explant de peau.

La figure 7 illustre l'implantation d'un sphéroïde en agarose « low melting » 1%.

La figure 9 illustre l'implantation d'un sphéroïde en acide hyaluronique 10 mg/ml.

## Revendications

1. Un procédé *in vitro* pour implanter des sphéroïdes dans un explant de peau, ledit procédé comprenant une étape c) d'implantation, au sein dudit explant de peau, d'au moins un sphéroïde, enrobé de préférence d'une matrice biologiquement dégradable.

2. Le procédé selon la revendication 1, **caractérisé en ce qu'**il comprend préalablement à l'étape c), une étape b) d'enrobage dudit au moins un sphéroïde dans une matrice biologiquement dégradable.

3. Le procédé selon la revendication 1 ou 2, **caractérisé en ce que** la matrice biologiquement dégradable est choisie dans le groupe comprenant ou consistant en la gélatine, les collagènes, les glycosaminoglycanes et les protéoglycanes de la matrice extracellulaire, l'élastine, les polysaccharides, une matrice synthétique biocompatible ou leur mélange, de préférence la matrice biologiquement dégradable est la gélatine, un glycosaminoglycane (ex. acide hyaluronique) ou un polysaccharide (ex. agarose).

4. Le procédé selon l'une quelconque des revendications 1 à 4, **caractérisé en ce qu'**il comprend, en préalable à l'étape c), et de l'étape b), une étape a) de coloration dudit au moins un sphéroïde, laquelle étape de coloration utilise un colorant choisi dans le groupe comprenant ou consistant en une sonde colorante ou une sonde fluorescente.

5. Le procédé selon l'une quelconque des revendications 1 à 4, **caractérisé en ce qu'**il comprend en outre une étape d) de mise en culture de l'explant après implantation dudit au moins un sphéroïde au terme de l'étape c), et ceci de préférence jusqu'à dégradation de la matrice biologiquement dégradable.

6. Le procédé selon l'une quelconque des revendications 1 à 5, **caractérisé en ce que** ledit au moins un sphéroïde est constitué de cellules tumorales et **en ce qu'**il vise à modéliser une tumeur de la peau.

7. Le procédé selon la revendication 6, **caractérisé en ce qu'**il vise à identifier des composés présentant un effet anti-cancéreux et **en ce qu'**il comprend en outre les étapes de :
e) Mise en contact d'au moins un composé avec un explant à l'issue de l'étape d),
f) Mise en culture du mélange issu de l'étape e), et
g) Identification des composés présentant un effet anti-cancéreux à l'issu de l'étape f).

8. Le procédé selon l'une quelconque des revendications 1 à 5, **caractérisé en ce que** ledit au moins un sphéroïde est constitué de cellules de follicules pileux, de glandes sébacées, de glandes sudoripares ou de corpuscules sensoriels et **en ce qu'**il vise à l'obtention d'échantillon pileux.

9. Un explant de peau, de préférence en culture, contenant au moins un sphéroïde obtenu par le procédé selon l'une quelconque des revendications 1 à 8.

10. L'explant de peau selon la revendication 9, **caractérisé en ce que** ledit au moins un sphéroïde est constitué de cellules tumorales ou de cellules de follicule pileux, de glandes sébacées, de glandes sudoripares ou de corpuscules sensoriels.

11. Utilisation de l'explant de peau selon la revendication 10 implanté d'au moins un sphéroïde constitué de cellules tumorales ou de cellules de follicule pileux, de glandes sébacées, de glandes sudoripares ou de corpuscules sensoriels pour l'identification de composés présentant un effet anti-cancéreux ou pour l'obtention d'échantillon pileux respectivement.

12. Un kit de mise en oeuvre du procède selon l'une quelconque des revendications 1 à 8 comprenant des cellules à même de former un sphéroïde, une matrice biologiquement dégradable, un explant de peau et, éventuellement, un dispositif d'implantation d'un sphéroïde.

## Patentansprüche

1. *In-vitro*-Verfahren zur Implantation von Sphäroiden in ein Hautexplantat, wobei das genannte Verfahren einen Schritt c) der Implantation mindestens eines Sphäroiden ins Innere des genannten Hautexplantats umfasst, der vorzugsweise mit einer biologisch abbaubaren Matrix umhüllt ist.

2. Verfahren nach Patentanspruch 1, **dadurch gekennzeichnet, dass** es vor dem Schritt c) einen Schritt b) der Umhüllung des genannten mindestens einen Sphäroiden mit einer biologisch abbaubaren Matrix umfasst.

3. Verfahren nach Patentanspruch 1 oder 2, **dadurch gekennzeichnet, dass** die biologisch abbaubare Matrix aus der Gruppe gewählt wird, umfassend oder bestehend aus Gelatine, Kollagenen, Glykosaminoglykanen und Proteoglykanen der extrazellulären Matrix, Elastin, Polysacchariden, synthetischen biokompatiblen Matrizen oder deren Gemischen, wobei die biologisch abbaubare Matrix vorzugsweise aus Gelatine, einem Glykosaminoglykan (beispielsweise Hyaluronsäure) oder einem Polysaccharid (beispielsweise Agarose) besteht.

4. Verfahren nach irgendeinem der Patentansprüche 1 bis 3, **dadurch gekennzeichnet, dass** es vor dem Schritt c) und dem Schritt b) einen Schritt a) der Färbung des genannten mindestens einen Sphäroiden umfasst, wobei der genannte Färbeschritt einen Farbstoff verwendet, gewählt aus der Gruppe, umfassend oder bestehend aus einer färbenden oder einer fluoreszierenden Sonde.

5. Verfahren nach irgendeinem der Patentansprüche 1 bis 4, **dadurch gekennzeichnet, dass** es außerdem einen Schritt d) der Kultur des Explantats nach Implantation des genannten mindestens einen Sphäroiden im Anschluss an den Schritt c) umfasst, und zwar vorzugsweise bis zum Abbau der biologisch abbaubaren Matrix.

6. Verfahren nach irgendeinem der Patentansprüche 1 bis 5, **dadurch gekennzeichnet, dass** der genannte mindestens eine Sphäroid aus Tumorzellen besteht und dadurch, dass es die Modellisierung eines Hauttumors beabsichtigt.

7. Verfahren nach Patentanspruch 6, **dadurch gekennzeichnet, dass** es beabsichtigt, Verbindungen zu identifizieren, die eine Krebs-hindernde Wirkung aufweisen und dadurch, dass es außerdem die folgenden Schritte umfasst:
e) Kontaktieren mindestens einer Verbindung mit dem Explantat im Anschluss an den Schritt d),
f) Kultur des aus dem Schritt e) hervorgegangenen Gemisches, und
g) Identifizieren der Verbindungen, die eine Krebs-hindernde Wirkung aufweisen, im Anschluss an den Schritt f).

8. Verfahren nach irgendeinem der Patentansprüche 1 bis 5, **dadurch gekennzeichnet, dass** der genannte mindestens eine Sphäroid aus Zellen von Haarfollikeln, Talgdrüsen, Schweißdrüsen oder Sinneskörperchen besteht und dadurch, dass es die Erzeugung von Haarproben beabsichtigt.

9. Hautexplantat, vorzugsweise in Kultur, mindestens einen Sphäroiden enthaltend, der nach dem Verfahren nach irgendeinem der Patentansprüche 1 bis 8 erzeugt wurde.

10. Hautexplantat nach Patentanspruch 9, **dadurch gekennzeichnet, dass** der genannte mindestens eine Sphäroid aus Tumorzellen oder aus Zellen von Haarfollikeln, Talgdrüsen, Schweißdrüsen oder Sinneskörperchen besteht

11. Gebrauch des Hautexplantates nach Patentanspruch 10, mit mindestens einem Sphäroiden aus Tumorzellen oder aus Zellen von Haarfollikeln, Talgdrüsen, Schweißdrüsen oder Sinneskörperchen implantiert, zum Identifizieren von Verbindungen, die eine Krebs-hindernde Wirkung aufweisen, bzw. zur Erzeugung einer Haarprobe.

12. Kit zur Ausführung des Verfahrens nach irgendeinem der Patentansprüche 1 bis 8, zur Ausbildung eines Sphäroiden geeignete Zellen umfassend, eine biologisch abbaubare Matrix, ein Hautexplantat und gegebenenfalls eine Vorrichtung zur Implantation eines Sphäroiden.

## Claims

1. An *in vitro* method for implanting spheroids in a skin explant, said method comprising a step c) of implanting at least one spheroid within said skin explant, preferably a spheroid coated with a biologically degradable matrix

2. The method according to claim 1, **characterized in that** it comprises, prior to step c), a step b) of coating said at least one spheroid in a biologically degradable matrix

3. The method according to claim 1 or 2, **characterized in that** the biologically degradable matrix is chosen from the group comprising or consisting of gelatin, collagens, glycosaminoglycans and proteoglycans of the extracellular matrix, elastin, polysaccharides, a biocompatible synthetic matrix or mixture thereof, preferably the biologically degradable matrix is gelatin, a glycosaminoglycan (e.g. hyaluronic acid) or a polysaccharide (e.g. agarose).

4. The method according to any one of claims 1 to 3, **characterized in that** it comprises, prior to step c), and step b), a step a) of coloring said at least one spheroid, which staining step uses a dye selected from the group comprising or consisting of a dye probe or a fluorescent probe.

5. The method according to any one of claims 1 to 4, **characterized in that** it further comprises a step d) of culturing the explant after implantation of said at least one spheroid at the end of step c ), and this preferably until degradation of the biologically degradable matrix.

6. The method according to any one of Claims 1 to 5, **characterized in that** the said at least one spheroid consists of tumor cells and **in that** it aims to model a skin tumor.

7. The method according to claim 6, **characterized in that** it aims to identify compounds exhibiting an anti-cancer effect and **in that** it further comprises the steps of:
e) Contacting at least one compound with an explant at the end of step d),
f) Culturing the mixture resulting from step e), and
g) Identifying the compounds exhibiting an anti-cancer effect at the end of step f).

8. The method according to any one of claims 1 to 5, **characterized in that** said at least one spheroid consists of cells from hair follicles, from sebaceous glands, from sweat glands or from sensory corpuscles and **in that** it aims obtaining a hair sample.

9. A skin explant, preferably in culture, containing at least one spheroid obtained by the method according to any one of claims 1 to 8.

10. The skin explant according to claim 9, **characterized in that** said at least one spheroid consists of tumor cells or of cells from hair follicle, from sebaceous glands, from sweat glands or from sensory corpuscles.

11. Use of the skin explant according to claim 10 implanted with at least one spheroid consisting of tumor cells or cells from hair follicle, from sebaceous glands, from sweat glands or from sensory corpuscles for the identification of compounds having a anti-cancer effect or for obtaining hair sample respectively.

12. A kit for implementing the method according to any one of claims 1 to 8 comprising cells capable of forming a spheroid, a biologically degradable matrix, a skin explant and, optionally, a device for implanting a spheroid .
